# NEUE EUROPÄISCHE PATENTSCHRIFT

(11) **EP 0 186 616 B2**
(45) Veröffentlichungstag und Bekanntmachung des Hinweises auf die Entscheidung über den Einspruch: **04.01.1995**
(45) Hinweis auf die Patenterteilung: 26.09.1990
(21) Anmeldenummer: 85730153.5
(22) Anmeldetag: 21.11.1985
(51) Int. Cl.: A61K 49/00

(54) **Magnetische Partikel für die Diagnostik**
Magnetic particles for diagnostic purposes
Particules magnétiques comme agent diagnostique

(30) Priorität: 23.11.1984 DE 3443251; 23.11.1984 DE 3443252; 04.03.1985 DE 3508000
(43) Veröffentlichungstag der Anmeldung: 02.07.1986
(73) Patentinhaber: SCHERING AKTIENGESELLSCHAFT, 13342 Berlin (DE)
(72) Erfinder: Gries, Heinz, Dr., D-1000 Berlin 31 (DE); Mützel, Wolfgang, Dr., D-1000 Berlin 45 (DE); Zurth, Christian, Dr., D-1000 Berlin 28 (DE); Weinmann, Hanns-Joachim, Dr., D-1000 Berlin 41 (DE)
(74) Vertreter: Lederer, Franz, Dr.

(56) Entgegenhaltungen:
- EP-A- 071 564
- EP-A- 130 934
- WO-A-78/00005
- WO-A-83/01738
- WO-A-83/03426
- WO-A-83/03920
- WO-A-84/02643
- WO-A-85/02772
- WO-A-85/04330
- WO-A-88/00060
- US-A- 3 832 457
- US-A- 3 932 805
- US-A- 4 001 288
- US-A- 4 018 886
- US-A- 4 101 435
- US-A- 4 247 406
- US-A- 4 335 094
- US-A- 4 452 773
- US-A- 4 770 183
- JOURNAL OF PHARMACEUTICAL SCIENCES, Band 68, Nr. 1, Januar 1979, Seiten 79-81, American Pharmaceutical Association, US; K. WIDDER et al.: "Magnetic microspheres: Synthesis of a novel parenteral drug carrier"
- CHEMICAL ABSTRACTS, Band 99, Nr. 7, August 1983, Seite 232, Nr. 49843k, Columbus, Ohio, US; V.M. RUNGE et al.: "Work in progress: potential oral and intravenous paramagnetic NMR contrast agents", & RADIOLOGY (EASTON, Pa.) 1983, 147(3), 789-91
- Cox, J. Pharm. Pharmacol. 24: 513-517 (1972)
- Ohgushi, J. Magn. Res. 29: 599-601 (1978)
- Lauterbur, "Frontiers of Biological Energetics" Ed. Dutton, Academic Press, NY, 1978, pp 752-759
- Lauterbur, Nature 242: 190-191 (1973)
- Tanaka, Proc. IEEE 66: 1582-1583 (1978)
- Tanaka, Sogo Rinsho 30 (1981)
- Abe, Radiation Medicine 2: 1-23 (1984)

## Beschreibung

Die Erfindung betrifft untenstehend im einzeln definierte Mittel zur Anwendung in der Diagnostik, die magnetische Partikel enthalten, bestehend aus einem magnetischen Doppelmetalloxid/-hydroxid und einem Komplexbildner.

Als magnetischer Bestandteil kommen magnetische Eisenoxide wie γ-Fe₂O₃ und Doppeloxide/Doppelhydroxide in Betracht, die zwei- und/oder dreiwertiges Eisen enthalten, wie beispielsweise Ferrite der allgemeinen Formel mMO · nFe₂O₃, worin M ein zweiwertiges Metallion oder ein Gemisch aus zwei zweiwertigen Metallionen ist, oder beispielsweise Ferrite der allgemeinen Formel nFeO · mM₂O₃, worin M ein dreiwertiges Metallion ist, und m und n die Zahlen 0, 1 bis 6 bedeuten. Bevorzugt sind Doppeloxide/Doppelhydroxide, die die physiologisch in geringen Mengen akzeptablen Elemente Magnesium, Zink, Eisen und Kobalt, gegebenenfalls auch noch in sehr geringen Mengen Mangan, Nickel, Kupfur, Barium und Strontium bzw. im Falle der dreiwertigen Ionen Chrom, Lanthan, Gadolinium, Europium, Dysprosium, Holmium, Ytterbium und Samarium enthalten.

Als phsiologisch verträgliche Komplexbildner können, Oligo- und Polysaccharide, verwendet werden. Bevorzugt sind, Dextrane, und Dextrine, an die gewünschtenfalls Biomoleküle geknüpft sind. Solche Biomoleküle können beispielsweise Hormone, wie Insulin, Prostaglandine, Steroide, sowie Aminozucker, Peptide, Protein oder Lipide sein, Besonders hervorzuheben sind Konjugate mit Albuminen, wie Humanserumalbumin, Staphylokokken-Protein A, Antikorpern, wie zum Beispiel monoklonale Antikörper und Konjugate oder Einschlußverbindungen mit Liposomen, die beispielsweise als unilamellare oder multilamellare Phosphatidylcholin-Cholesterol-Vesikel eingesetzt werden.

Die zur Anwendung kommenden Komplexbildner bzw. Stabilisatoren sollen eine Trennung von Magnetteilchen und Flüssigkeit verhindern. Hierfür müssen die Mangetteilchen mit einer Schicht von langkettigen Molekülen bedeckt sein, die mehr oder weniger senkrecht zur Oberfläche in den Raum orientiert sind. Bei adsorptionsstabilisierten magnetischen Flüssigkeiten ist der polare Teil des Stabilisatormoleküls mit der Oberfläche des magnetischen Teilchens über die elektrostatische Welchselwirkung verbunden, bei chemisch stabilisierten magnetischen Flüssigkeiten sind die Stabilisatormoleküle chemisch an die Teilchenoberfläche gebunden. Diese chemische Bindung kann z.B. in der Weise erfolgen, wie sie in der DDR-Patentschrift 160532 offengelegt ist. Für die Anwendung in der NMR-Diagnostik soll im allgemeinen die durchschnittiche Teilchengröße für die Metallpartikel weniger als 500 Å, für die Ferrite weniger als 150 Å im Durchmesser betragen. Komplexe von Magnetit (Fe₃O₄) mit Dextran sind zum Beispiel beschrieben in den US-Patenten 4.101.435 und 4.452.773. Sie bilden in Wasser stabile Sole, die aufgrund ihrer magnetischen Eigenschaften vielfältige Verwendung finden können. So sind sie unter anderem als Drug-Carrier (vor allem für Cytotoxika bei der Tumorbehandlung), als Agens zur Messung des Blutstroms, als Marker in der Scanning-/transmission-Elektronenmikroskopie, zur Kennzeichnung und Abtrennung von Zellen und Biomolekülen (zum Beispiel eines Antigens aus einer Antigenmischung, indem man Partikel benutzt, die kovalent an den entsprechenden Antikörper gebunden sind), sowie auch zur Anwendung auf mechanischem Gebiet (z.B. für Ton- und Videobänder) geeignet. Ferner ist Dextran-Magnetit als Relaxationsagens zur Messung des Wasseraustausches an Erythrozytenmembranen vorgeschlagen worden (Biochem. and Biophys. Res. Comm. *97*, 114 (1980).

Viele der bischer beschriebenen magnetischen Flüssigkeiten sind für eine diagnostische Anwendung ungeeignet da sie physiologisch unverträgliche Komponenten enthalten.

Es wurde nun gefunden, daß die enfindungsgemäßen Mittel überraschenderweise die vielfältigen Voraussetzungen für die Eingung als Kontrastmittel für die NMR-Diagnostik erfüllen. (Eine ausführliche Diskussion dieser Voraussetzungen findet sich in der Europäischen Patentanmeldung Publikations Nr. EP-A-71 564 und der Deutschen Patentanmeldung DE-A-34 01 052.1).

So sind sie hervorragend dazu geeignet, nach enteraler oder parenteraler Applikation durch Veränderung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Kontrastmitteln zu belasten, und die gute Verträglichkeit, die notwendig ist, um den nichtinvasiven Charakter der Untersuchung aufrechtzuerhalten.

Als besonders günstig ist hierbei anzusehen, wenn als Träger der magnetischen Eigenschaften des Eisen fungiert, ein physiölogisch unbedenkliches Element, das für den menschlichen Organismus sogar essentiell ist. Da überraschenderweise im Vergleich zu allen vorbekannten Kontrastmitteln die wirksame Dosis außerordentlich niedrig ist, ergibt sich ein sehr hoher Sicherheitsabstand für die Verwendung der Komplexe in vivo.

Die gute Wasserlöslichkeit der erfindungsgemäßen Mittel erlaubt es, hochkonzentrierte Lösungen herzustellen, um die Volumenbelastung des Kreislaufs in vertretbaren Grenzen zu halten und die Verdünnung durch die Körperflüssigkeit auszugleichen. Weiterhin weisen die erfindungsgemäßen Mittel nicht nur eine hohe Stabilität in vitro, sondern auch eine überraschend hohe Stabilität in vivo auf.

Ein besonderer Vorzug der erfindungsgemäßen Mittel ist es, daß mit ihnen aufgrund spezifischer pharmakokinetischer Eigenschaften Gewebe, Organe und Organsystem in ihrer Signalintensität im Kernspintomogramm stark verändert werden können. Zum ersten Mal stehen gut verträgliche Kontrastmittel u.a. für die bildliche Darstellung von Tumoren der Leber und Milz zur Verfügung. Durch Bindung des ferromagnetischen Materials an Biomoleküle wie zum Beispiel monoklonale für tumorassoziierte Antigene spezifische Antikörper oder Anti-Myosin, gelingt eine Verbesserung der Tumor- und Infarkt-Diagnostik. Als monoklonale Antikörper für die Konjugation kommen insbesondere solche infrage, die gegen überweigend zellmembranständige Antigen gerichtet sind. Als solche sind zum Beispiel für die Tumordarstellung monoklonale Antikörper bzw. deren Fragmente (F(ab)₂) geeignet, die zum Beispiel gegen das Carcinoembryonale Antigen (CEA), humanes Choriogonadotropin (β-hCG) oder andere tumorständige Antigene, wie Glycoproteine, gerichtet sind. Geeignet sind u.a. auch Anti-Myosin-, Anti-Insulin- und Anti-Fibrin-Antikörper.

Konjugate oder Einschlußverbindungen mit Liposomen eignen sich für Leberuntersuchungen. Die NMR-Diagnostik im Magen-Darm-Bereich wird durch enterale Applikation der erfindungsgemäßen Mittel verbessert, wodurch beispielsweise eine bessere Abgrenzung von Darmabschnitten bei Pakreas-Untersuchungen erreicht wird.

Die Herstellung, der Mikrosuspensionen der Doppelmetall-oxid/-hydroxid-Komplexe erfolgt in an sich bekannter Weise dadurch, daß man wäßrige Lösungen der entsprechenden zwei- und dreiwertigen Metallsalze, beispielsweise die Halogenide, zusammengibt. Dann wird mit Alkalihydroxiden, wie zum Beispiel Ammonium- oder Natriumhydroxid und/oder Alkalicarbonaten, wie zum Beispiel Natriumcarbonat versetzt, um den pH-Wert zu erhöhen und die Metalloxide bzw. Metallhydroxide in Form feinster Partikel zu erzeugen, an die der Komplexbildner bindet. Durch zum Beispiel Zentrifugieren sowie zum Beispiel Gelfiltrations-Chromatographie und/oder Dialyse kann eine Abtrennung und Reinigung der gewünschten Komplexe erfolgen.

In einer anderen Herstellungsweise wird das fein gemahlene Doppeloxid bzw. Metall mit dem Schutzkolloid behandelt (siehe zum Beispiel J. Pharm. Sci. *68*, 79, (1979).

Die Bindung der Biomoleküle erfolgt in an sich bekannter Weise nach Methoden, wie sie zum Beispiel in Rev. roum. Morphol. Embryol. Physiol., Physiologie 1981, *18*, 241 und J. Pharm. Sci. *68*, 79, (1979) beschrieben sind.

Die Herstellung der erfindungsgemäßen diagnostischen Mittel erfolgt ebenfalls in an sich bekannter Weise, indem man die erfindungsgemäßen Partikel gegebenenfalls unter Zugabe der in der Galenik üblichen Zusätze in wäßrigem Medium suspendiert und anschließend die Suspension gegebenenfalls sterilisiert. Geeignete Zusatze sind beispielsweise physiologisch unbedenkliche Puffer (wie z.B. Tromethamin) oder, falls erforderlich, Elektrolyte wie zum Beispiel Natriumchlorid oder falls erforderlich Antioxidantien wie zum Beispiel Ascorbinsäure.

Sind für die enterale Verabreichung oder andere Zwecke Suspensionen der erfindungsgemäßen Mittel in Wasser oder physiologischer Salzlösung erwünscht, werden sie mit einem oder mehreren in der Galenik üblichen Hilfsstoffen (z.B. Methylcellulose, Lactose, Mannit) und/oder Tensiden (z.B. Lecithine, Tweens®, Myrj®) und/oder Aromastoffen zur Geschmackskorrektur (z.B. ätherischen Ölen) gemischt.

Die erfindungsgemäßen diagnostischen Mittel enthalten 1 µMol bis 1 Mol, vorzugsweise 0,1 bis 100 mMol magnetisches Metall pro Liter und werden in der Regel in Mengen von 0,001 bis 100 µMol, vorzugsweise 0,1 bis 10 µMol magnetisches Metall pro Kilogramm Körpergewicht dosiert. Sie sind zur enteralen und parenteralen Applikation bestimmt.

Die folgenden Ausführungsbeispiel dienen zur weiteren Erläuterung der Erfindung.

### Beispiel 1

80 g Dextrin (Polymaltose, basale Viskosität 0,05/25°C) werden in 180 ml Wasser bei 70°C in Lösung gebracht. Nach Abkühlen auf Raumtemperatur wird in eine Mischung aus 70 ml 1-molarer Eisen-III-chloridlösung und 35 ml einer 1-molaren Eisen-II-chloridlösung eingerührt. Dann bringt man die Mischung durch tropfenweise Zugabe einer 20 gewichts-%igen wäßrigen Natriumcarbonatlösung auf pH 1,7. Nach Beendigung der Gasentwicklung stellt man durch tropfenweise Zugabe von 10 n-Natronlauge einen pH-Wert von 11,0 ein und erhitzt 30 Minuten zum Rückfluß. Nach Abkühlen auf Raumtemperatur bringt man durch Zugabe von 6-normaler Salzsäure auf pH 6,2, fällt den Komplex durch Zugabe von 500 ml Ethanol, zentrifugiert, löst den Rückstand in Wasser und entfernt Fremdionen durch Dialyse. Die kolloide Lösung wird nach Filtration lyphilisiert. Man erhält den gewünschten Dextrin-Magnetit-Komplex als schwarzes Pulver.

### Beispiel 2

112 mg Dextrin-Magnetit-Komplex (Beispiel 1) werden in 20 ml einer 0,9%igen Kochsalzlösung eingetragen. Das 15 Minuten bei 110°C pasteurisierte Sol dient zur parenteralen Applikation.

### Beispiel 3

Ein Granulat, hergestellt aus 12 mg Dextrin-Magnetit-Komplex (Beispiel 1), 2,42 g Tromethamin, 45 g Mannit und 10 g Tylose, wird in 1000 ml Aqua dest. eingerührt für die enterale Applikation verwendet.

### Beispiel 4

112 mg Dextran-Magnetit-Komplex (bezogen von Fa. Meito Sangyo, Japan) werden unter Rühren in 20 ml einer 0,9%igen Kochsalzlösung eingetragen. Das erhaltene So wird in Ampullen abgefüllt und hitzesterilisiert.

### Beispiel 5

Ein Granulat, hergestellt aus 12 mg Dextran-Magnetit-Komplex, (bezogen von Fa. Meito Sangyo, Japan) 2,42 g Tromethamin, 45 g Mannit und 10 g Tylose, wird in 1000 ml Aqua dest. eingerührt für die enterale Applikation verwendet.

### Beispiel 6

Man mischt 40 ml einer 1-molaren Eisen-III-chlorid-Lösung und 20 ml einer 1-molaren Zinkchlorid-Lösung und erwärmt auf 80°C. Die heiße Lösung geißt man unter intensivem Rühren in eine Lösung von 6,8 g Natriumhydroxid in 28 ml Wasser. Man hält 24 Stunden am Rückfluß, zentrifugiert die Suspension nach Abkühlen auf Raumtemperatur, suspendiert den Rückstand in 100 ml Wasser und bringt die Suspension mit konzentrierter Salzsäure auf pH 1,4. Man löst 18 g Dextran T 10 (Pharmacia) in 100 ml Wasser und erhitzt nach Zugabe von 1,8 ml 40 %iger Natronlauge eine Stunde zum Rückfluß. Nach Abkühlen auf Raumtemperatur versetzt man die neutrale Lösung mit 1000 ml Methanol. Nach Stehen über Nacht wird vom wäßrigen Methanol abdekantiert und der Rückstand in 100 ml Wasser gelöst. Zu dieser Lösung gibt man die Zinkferritsuspension und erhitzt die Mischung unter intensivem Rühren 40 Minuten zum Rückfluß. Nach dem Abkülen wird die kolloide Lösung neutralisiert und durch Dialyse ionenfrei gemacht. Nach Lyophilisieren erhält man den Dextran-ZnO · Fe₂O₃-Komplex als braunes Pulver. In analoger Weise wird unter Verwendung einer 1-molarer Bariumchlorid-Lösung ein Dextran-Bariumferrit-Komplex als braunes Pulver erhalten.

### Beispiel 7

Der in Beispiel 6 erhaltene Dextran-Zinkferrit-Komplex wird in Multivials eingefüllt. Nach Zugabe von physiologischer Kochsalzlösung erhitzt man 20 Minuten auf 120°C. Man erhält eine gebrauchsfertige sterilisierte kolloide Injektionslösung.

## Patentansprüche

1. Verwendung magnetischer Partikel, die Doppelmetall-oxid/hydroxid und Komplexbildner enthalten, wobei die Komplexbildner alkalibehandelte Oligo- oder Polysaccharide sind, zur Herstellung von Kontrastmitteln für die NMR Diagnostik.

2. Verwendung gemäß Patentanspruch 1, dadurch gekennzeichnet, daß das Doppelmetall-oxid-/-hydroxid ein Ferrit der allgmeinen Formel mMO · nFe₂O₃, worin M ein zweiwertiges Metallion und m und n die Zahlen 0, 1 bis 6 darstellen, ist.

3. Verwendung gemäß Patentanspruch 1, dadurch gekennzeichnet, daß das Doppelmetall-oxid-/hydroxid die allgemeine Formel nFeO · mM₂O₃, worin M ein dreiwertiges Metallion und m und n die Zahlen 0, 1 bis 6 darstellen, hat.

4. Verwendung gemäß Patentanspruch 1, dadurch gekennzeichnet, daß der Komplexbildner Dextran oder Dextrin ist.

5. Verwendung gemäß Patentanspruch 1, dadurch gekennzeichnet, daß die Partikel Magnetit, Bariumferrit oder Zinkferrit enthalten.

6. Verwendung gemäß Patentanspruch 1, dadurch gekennzeichnet, daß die Partikel Dextran-Magnetit enthalten.

7. Verwendung gemäß Patentanspruch 1, dadurch gekennzeichnet, daß die Partikel Dextrin-Magnetit enthalten.

8. Verwendung gemäß Patentanspruch 1, dadurch gekennzeichnet, daß die Partikel Dextran-Zinkferrit enthalten.

9. Verwendung gemäß Patentanspruch 1, dadurch gekennzeichnet, daß die Partikel Dextran-Bariumferrit enthalten.

10. Verwendung gemäß einem der Patentansprüche 1-9, dadurch gekennzeichnet, daß die Kontrastmittel pro Liter 1 µMol bis 1 Mol magnetisches Metall enthalten.

## Claims

1. The use of magnetic particles that contain a double metal-oxide/hydroxide and a complexing agent, wherein the complexing agent comprises alkali-treated oligo- or polysaccharide, for the preparation of contrast agents for NMR diagnostics.

2. The use according to patent claim 1, characterised in that the double metal-oxide/hydroxide is a ferrite of the general formula mMO.nFe₂O₃, wherein M is a divalent metal ion and m and n represent the numbers 0 or 1 to 6.

3. The use according to patent claim 1, characterised in that the double metal-oxide/hydroxide is of the general formula nFeO.mM₂O₃, wherein M represents a trivalent metal ion and m and n represent the numbers 0 or 1 to 6.

4. The use according to patent claim 1, characterised in that the complexing agent is dextran or dextrin.

5. The use according to patent claim 1, characterised in that the particles contain magnetite, barium ferrite or zinc ferrite.

6. The use according to patent claim 1, characterised in that the particles contain dextran-magnetite.

7. The use according to patent claim 1, characterised in that the particles contain dextrin-magnetite.

8. The use according to patent claim 1, characterised in that the particles contain dextran-zinc ferrite.

9. The use according to patent claim 1, characterized in that the particles contain dextran-barium ferrite.

10. The use according to any one of patent claims 1 to 9, characterised in that the contrast agents contain from 1 µmole to 1 mole of magnetic metal per litre.

## Revendications

1. Utilisation de particules magnétiques qui contiennent des oxydes/hydroxydes doubles de métaux et des générateurs de complexes, les générateurs de complexes étant des oligo- ou polysaccharides traités par des matières alcalines, pour la préparation d'agents de contraste pour le diagnostic par RMN.

2. Utilisation selon la revendication 1, caractérisée en ce que l'oxyde/hydroxyde double de métaux est une ferrite de formule générale mMO.nFe₂O₃, dans laquelle M est un ion de métal divalent et m et n représentent les nombres 0 et 1 à 6.

3. Utilisation selon la revendication 1, caractérisée en ce que l'oxyde/hydroxyde double de métaux a la formule générale nFeO.mM₂O₃, dans laquelle M est un ion de métal trivalent et m et n représentent les nombres 0 et 1 à 6.

4. Utilisation selon la revendication 1, caractérisée en ce que le générateur de complexes est du dextrane ou de la dextrine.

5. Utilisation selon la revendication 1, caractérisée en ce que les particules contiennent de la magnétite, de la ferrite de baryum ou de la ferrite de zinc.

6. Utilisation selon la revendication 1, caractérisée en ce que les particules contiennent du dextrane-magnétite.

7. Utilisation selon la revendication 1, caractérisée en ce que les particules contiennent de la dextrine-magnétite.

8. Utilisation selon la revendication 1, caractérisée en ce que les particules contiennent du dextrane-ferrite de zinc.

9. Utilisation selon la revendication 1, caractérisée en ce que les particules contiennent du dextrane-ferrite de baryum.

10. Utilisation selon l'une des revendications 1 à 9, caractérisée en ce que les agents de contraste contiennent, par litre, 1 µmole à 1 mole de métal magnétique.
